# EUROPEAN PATENT APPLICATION

(11) **EP 2 072 074 A1**
(43) Date of publication of application: **24.06.2009**
(21) Application number: 08021873.8
(22) Date of filing: 17.12.2008
(51) Int. Cl.: A61M 16/00, A61M 16/04

(54) **Tracheostomy device**

(30) Priority: 19.12.2007 US 14848 P
(71) Applicant: Isla, Roger, Steubenville OH 43952 (US)
(72) Inventor: Isla, Roger, Steubenville OH 43952 (US)
(74) Representative: Lee, Nicholas John

(57) **Abstract**

A curved tube (2) having a proximal end (4), designed to allow insertion of tools and including a lip to prevent the tube from slipping out of a conventional tracheostomy tube, is provided herein. The curved tube (2) also has a distal end (3) that is pointed, sharpened, and beveled upwards. The distal end (3) of the device is useful for cutting the throat and trachea of a patient in need of a tracheostomy, allowing the insertion of the device into the trachea of the patient. The proximal end (4) of the device includes tubes of successively increasing size. Methods of performing a tracheostomy using the device are also provided herein.

## Description

CLAIM TO PRIORITY

This application claims priority to United States Provisional Patent Application No. 61/014,848, filed on December 19, 2007. That application is incorporated by reference as if fully rewritten herein.

BACKGROUND OF THE INVENTION

An otolaryngologist (head and neck surgeon) is often involved in the care of patients with upper respiratory obstruction, many of whom require tracheostomy, also known as tracheotomy. The terms are used interchangeably herein. Some tracheostomies are elective, others are performed as an emergency. An elective tracheostomy procedure may require approximately 45 minutes of operating room (OR) time. An emergency procedure may become necessary in a matter of seconds.

A problem arises when a patient needs an emergency tracheostomy and an ear, nose, and throat specialist (ENT) or other trained surgeon is not readily available. The best case scenario involves at least 20-30 minutes for an ENT to be available. Unfortunately, a patient deprived of oxygen for a maximum of five minutes will suffer irreversible brain damage or even death.

Tracheotomy as it is performed today was first described by Chevalier Jackson in 1909. It consisted of making a vertical or horizontal incision between the cricoid and sternal notch on the neck then dissecting and exposing the strap muscles and bridge of the thyroid. The muscles are retracted, exposing the anterior tracheal wall. A window is created by resecting a segment of a tracheal ring between the second or third space. A tracheotomy tube is then introduced.

In 1955 Sheldon described for the first time a modem alternative to the Jackson tracheotomy. Sheldon's method consisted of the percutaneous introduction of a tracheotomy tube loaded to a trocar. This technique produced a significant complication rate due to laceration of adjacent structures and was later abandoned.

In 1953 Seldinger, a radiologist, introduced the technique of guide wire needle replacement in percutaneous arterial catheterizations. This technique was the catalyst for the development of varied medical devices that were used and introduced via the guide wire including percutaneous tracheotomies. Since then, many percutaneous tracheotomies over a guide wire have been reported and gained acceptance and popularity worldwide.

In 1969 Toy and Weigstein developed a tapered straight dilator over a guiding catheter. In 1985 Ciaglia *et al*. reported a new technique of percutaneous dilation tracheostomy (PDT) using serial dilators over a guide wire. In 1989 Schackner *et al*. developed a single dilating tracheotomy forceps over a guide wire (Rapitrac). In 1990 Griggs developed another dilatation forceps over a guide wire for the performance of PDT. Finally, in 1997 Fantoni reported a non-surgical tracheotomy that was done by the translaryngeal route.

During the past 20 years one of the known percutaneous dilatation tracheotomy techniques was and is currently used to perform tracheotomies in hospitals across the globe. The technique is standard and very simple; it consists of identifying the second or third tracheal ring space and making a 1.5 cm vertical or horizontal incision, then dissecting down to the trachea and introducing a 14 bore needle in the trachea. A guide wire is passed through the needle, and the needle is removed. Serial dilators or a single tapered one are introduced with a forceps over the guide wire. Finally, the dilator is loaded with a tracheotomy tube and introduced into the tracheal lumen. This procedure is performed under endoscope guidance (most of the time) and under local anesthetic, usually on patients who are already intubated in the intensive care unit (ICU). This is a procedure typically done at the bedside.

The indications for percutaneous tracheotomies are the same as per the ones for conventional surgical tracheotomies: namely, patients requiring long term intubation, patients who need tracheal bronchial toilette, patients who need better management of their ventilatory activity, and patients with any condition that produces upper respiratory obstruction of a significant degree. Few contraindications apply to PDT. These include, for example, large goiters, anatomical deformities of the neck, and acute infection of the anterior neck.

The complications of PDTs mirror those for conventional surgical tracheotomies; however, some differences exist. Fracture of tracheal rings is a very common complication and it is almost unique for this procedure; therefore, late tracheal stenosis is a potential by-product that can be serious and difficult to correct. Fortunately the stenosis has to be severe (> 75%) before becoming symptomatic.

Accidental decannulation in the early post-op can be fatal because of the difficulty of reintroducing a tube through a small incision. Fatal puncture of the aorta and innominate artery have been reported.

It would be beneficial to develop a surgical device that could allow healthcare professionals to perform an emergency tracheostomy in a timely manner. The technique to be described can be done in a safe, fast manner with minimal risk once the technique is mastered and when the device is used. It would also be beneficial to develop a simple device that could allow a trained soldier or layperson to perform a tracheostomy in an emergency.

BRIEF SUMMARY OF THE INVENTION

Provided herein is an all-in-one puncture, knife, tracheal dilator, and tracheostomy tube inserter. Devices as claimed and described herein include, for example, a curved tube having a proximal end, designed to allow insertion of tools and including a lip to prevent the tube from slipping out of a conventional tracheostomy tube. The curved tube also has a distal end that is pointed, sharpened, and beveled upwards. The distal end of the device is useful for cutting the throat and trachea of a patient in need of a tracheostomy, allowing the insertion of the device into the trachea of the patient. The proximal end may have one or more additional integral tubes of increasing diameter. Although the interior and exterior diameters of the additional tubes may both increase relative to the curved tube and any intervening tubes, the exterior diameter may increase without a corresponding increase of the interior diameter.

A tracheostomy may be performed using devices as described herein. Such a tracheostomy may be an emergency tracheostomy in which the device is used alone and allowed to remain in the trachea, or it may be a tracheostomy in which the device is inserted into a patient's trachea while the device is situated in a conventional tracheostomy tube. After insertion of the device and the conventional tracheostomy tube, the device is removed from the patient, leaving the conventional tracheostomy tube for further use.

BRIEF DESCRIPTION OF THE SEVERAL VIEWS OF THE DRAWINGS

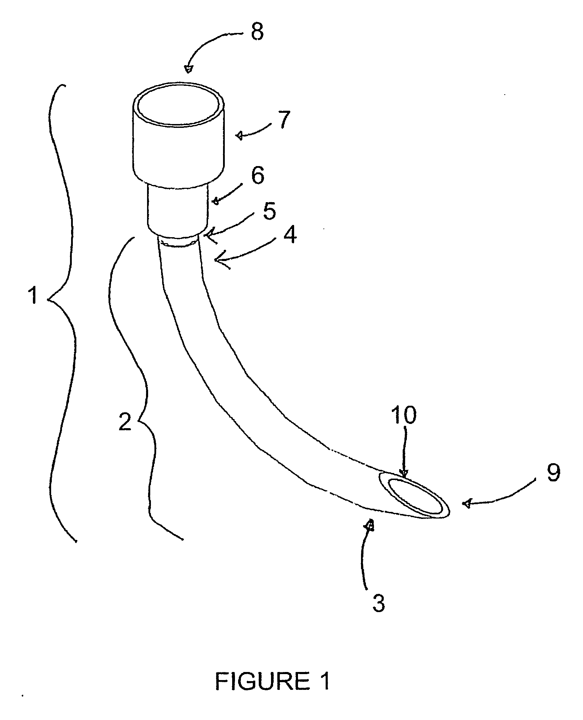

Figure 1 shows a perspective view of a device 1 according to one embodiment of the invention. The device 1 includes a tube 2 having a distal end 3 and a proximal end 4. The embodiment shown includes a beveled connector 5 intermediate the tube 2 and the transitional tube 6. The transitional tube 6 is intermediate the beveled connector 5 and the stopping end 7. It is this stopping end 7 that prevents the device from being pushed out of the end of a conventional trach tube when the device 1 is inserted while in a trach tube. Tube 2 further includes a proximal opening 8 and distal opening 9, both of which place the environment in communication with the interior of the tube 2. A cutting edge 10 is disposed on the distal end 3 of tube 2.

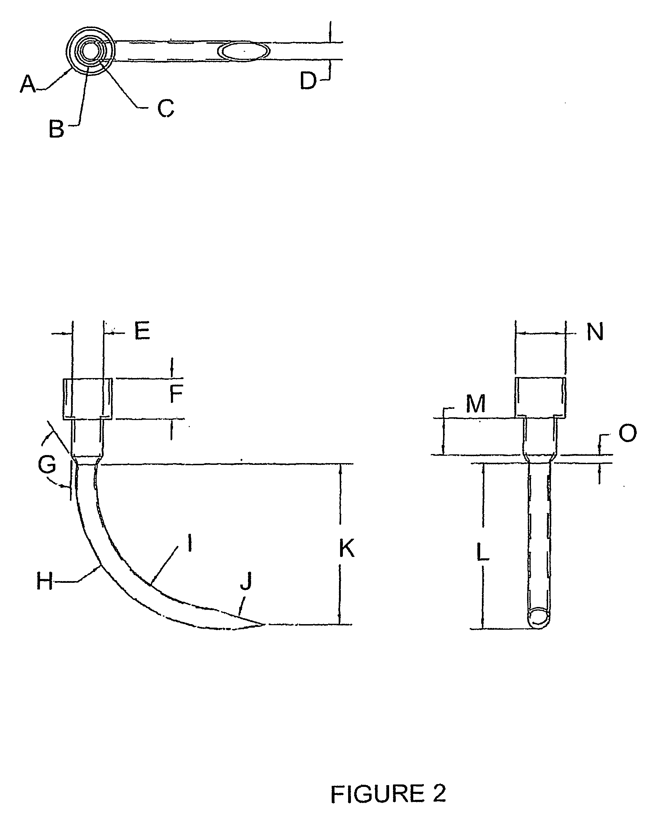

Figure 2 shows a top, side, and front view of a device 1 as taught herein. Although one skilled in the art will recognize that various embodiments of the invention may be various sizes, in one embodiment of the invention the sizes of the device are as set forth below. It should be noted, of course, that these sizes are exemplary, and that while certain embodiments may be of the same size, or may be of different size but have identical proportions, this recitation should not be interpreted to limit the claims unless, of course, the specific dimensions are recited in the claims. Although not indicated in the figure, the wall thickness of the tube 2 is consistently 0.04 inches. All of the exemplary dimensions are reported in inches, with the exception of the measurement noted in degrees:

| Reference Letter | Dimensions |
|---|---|
| A | Radius 0.36 |
| B | Radius 0.23 |
| C | Radius 0.15 |
| D | Length 0.25 |
| E | Length 0.46 |
| F | Length0.60 |
| G | Angle 146 degrees |
| H | Radius 2.22 |
| I | Radius 1.93 |
| J | Radius 9.60 |
| K | Length 2.36 |
| L | Length 2.43 |
| M | Length 0.55 |
| N | Length 0.71 |
| O | Length 0.12 |

DETAILED DESCRIPTION OF THE INVENTION

Various embodiments of a tracheostomy device are taught herein. Embodiments typically provide an all-in-one puncture, knife, tracheal dilator, and tracheostomy tube inserter. Devices may comprise, for example, a curved tube having a proximal end, designed to allow insertion of tools and including a lip to prevent the tube from slipping out of a conventional tracheostomy tube.

The proximal end includes a beveled connector, which is a tube, leading to a transitional tube. The transitional tube is connected to a stopping end, which is also a tube. Between the transitional tube and the stopping end there may be, but is not required to be, a second beveled connector. The diameter of the transitional tube is greater than the diameter of the curved tube, and is uniform throughout the transitional tube. The diameter of the stopping end is greater than the diameter of the transitional tube. The diameter of the beveled connector increases between that of the curved tube and that of the transitional tube.

The combination of tubes offers a number of benefits. For example, the stopping tube prevents the tracheostomy device from being pushed completely through a tracheostomy tube. It may also facilitate connection to a bag valve mask. Furthermore, the use of differing diameters may facilitate handling and insertion of the device.

The curved tube also has a distal end that is pointed, sharpened, and beveled upwards. The distal end of the device is useful for cutting the throat and trachea of a patient in need of a tracheostomy, allowing the insertion of the device into the trachea of the patient.

Typically, embodiments of the invention are tracheostomy devices that conform to the curvature and inner diameter of the conventional tracheostomy tubes currently available in the operating rooms in hospitals in the United States. It has the advantage that the tube itself can be used temporarily as a ventilation device attached to a bag valve mask (for example, an AMBU® bag) or an anesthesia machine. Of course, other acceptable sizes may be used. The device, when it is inserted in the lumen of a tracheostomy tube, typically protrudes beyond the tip of the tube. Preferably this protrusion is about 2.5 cm.

The tip of the device is beveled upward. Preferably the bevel is 30 degrees, though it may range between, for example, 20 degrees and 40 degrees. The tip is sharp and the borders of the bevel are sharp to allow cutting. The device may be any material suitable for surgical insertion. Typically it is a metal or plastic. In one embodiment it is copper.

Embodiments of the device may have a constant inner and/or outer diameter throughout the entire tube. Other embodiments may have an inner and/or outer diameter that decreases along the length of the tube. The amount of decrease may be uniform along the length of the device or not uniform. The decrease may only continue from the distal end of the device through about half of the device's length. The thickness of the wall of the device may be constant. The thickness may be constant throughout part of the length of the device and may vary throughout another part of the length.

The diameter of the device may transition to the conventional tracheostomy tube in a smooth fashion when the device is loaded in a tube. The entire device, including but not limited to the device's tip, may be solid and inflexible.

The shape of the instrument conforms to a standard tracheotomy tube such as a Shiley, therefore the dilating pressure is not exerted on an anterior-posterior direction as is the case with the other techniques but horizontally along the vertical axis of the trachea, preventing fracture of the tracheal rings or misplacement. In cases where the surgeon finds increased resistance to the introduction of the tube, one easily can use a standard tracheal retractor placed in the space of the bevel and pulling gently allowing an easy introduction of the tube.

Embodiments of the invention may be offered in different tracheal tube sizes and possibly to fit different brands, such as Portex, Bivona, or others. The device may be offered in a kit with other tracheal tubes and instructions for use.

Devices described herein may have a single interior passage. They may have a plurality of interior passages. Where a plurality of interior passages are provided, they may be of the same shape or different shapes, and of the same size or different sizes. The interior of a device may further include one or more grooves and/or connectors for situating, orienting, or connecting accessories to the device. Optical attachments can be added to this device. These may include, for example, rigid fiberoptic probes conforming to the curvature of the tube and attached to the video monitor device. This could be utilized as a teaching device as well.

The design of embodiments as presented herein takes into consideration the anatomy of the trachea and the neck, its slight anterior curvature, and its progressive deeper position as it descends into the mediastinum and its relationship with the great arterial structures in the lower neck. It should be noted that the cartilaginous rings of the trachea are really incomplete rings that resemble a horse-shoe. When anterior posterior pressure is exerted on them, they actually "ride" over the cervical vertebrae, bringing the posterior soft tracheal wall along with the esophagus into the lumen. This risks injury. The elasticity of the tracheal rings in pediatric patients in particular has remained a contraindication for PDTs, because the elasticity of the trachea increases risk of complications. On the other hand, the stiffness of the cartilages rings secondary to calcification in the elderly, makes them susceptible to fractures when applying anterior posterior pressure against the cervical vertebras.

Embodiments as presented herein are not meant to replace conventional tracheal tubes that are inserted into the trachea, but rather to act as a facilitator and a carrier of these tubes into the trachea lumen in a simple, one step, one instrument procedure in a safe and effective manner. Because the device has an interiorly placed bevel with a sharp tip and cutting borders, and a diameter similar to available tracheal tubes, it can, if desired, be introduced only partially and can be attached it to an AMBU® bag or an anesthesia machine in extreme emergency situations, until specialized help arrives. Therefore it would be very useful, for example, in ERs, in ambulances, at restaurants, in the battle field, etc. because it will save lives.

EXAMPLES

A vertical 1.5 cm incision is made two finger breadths from the sternal notch in the midline. Then the soft tissues are spread with standard scissors and a tracheostomy device as described herein is put through the incision down to the anterior tracheal wall. By moving the device up and down just slightly the surgeon can feel the inter-tracheal space and then apply pressure downward gently until air through the tube is heard or noticed. At this point additional pressure is applied until the tip of the tracheostomy tube is in the tracheal lumen. Next the tracheostomy device is removed and the remainder of the tracheal tube length advanced and fixed in place.

The whole procedure should take only two to three minutes. Should any questions of the proper placement of the tube arise during the procedure a flexible fiberoptic laryngoscope can be passed through the tracheostomy device to confirm the position. Once confirmed, removal of the scope and advancement of the tube can be completed.

The described technique has the advantage of streamlining the tracheostomy process by puncturing, cutting and dilating the trachea and introducing a loaded trach tube all with one single instrument. This technique does not require endoscopic guidance because it produces visual and auditory feedback when the instrument is inserted; it is easy to identify the tracheal spaces by sliding the tip of the instrument up and down over the tracheal rings. Because of its simplicity the procedure can be completed in about 2-3.

The procedure reported herein has a number of advantages over the prior art. For example, it can be performed under both elective and emergent conditions. It can be indicated in pediatric patients under 12. It can be easily done after watching an instructional video that will be provided with the kit or purchased independently through a website or after attending seminars arranged by the manufacturer.

## Claims

1. A tracheostomy device, comprising:
a curved tube having a distal end and a proximal end; and
a distal opening at the distal end and a proximal opening at the proximal end, wherein said distal opening comprises a beveled edge and a pointed tip, and wherein said beveled edge is disposed at the distal opening on the interior circumference of the curved tube, and the pointed tip is disposed on the exterior circumference of the curved tube;
a transitional tube at the proximal end of said curved tube; and
a beveled connector intermediate said curved tube and said transitional tube, wherein the exterior diameter of the beveled connector increases uniformly along its length from the same as the exterior diameter of the curved tube to the same as the exterior diameter of the transitional tube.

2. The tracheostomy device of claim 1, wherein the interior of said curved tube defines a single interior channel.

3. The tracheostomy device of claim 1, wherein the interior of said curved tube is partitioned into multiple channels.

4. The tracheostomy device of any preceding claim, wherein said curved tube has a wall thickness that is uniform throughout the length of the curved tube.

5. The tracheostomy device of any preceding claim, wherein said curved tube is rigid throughout its length.

6. The tracheostomy device of any preceding claim, wherein said transitional tube has an exterior diameter greater than the exterior diameter of the curved tube.

7. The tracheostomy device of any preceding claim, wherein said transitional tube has an interior diameter that is the same as the interior diameter of the curved tube.

8. The tracheostomy device of any preceding claim, further comprising a stopping end disposed on said transitional tube opposite said beveled connector, wherein the exterior diameter of the stopping end is greater than the exterior diameter of the transitional tube.

9. The tracheostomy device of any preceding claim, wherein said beveled edge is angled at between 20 and 40 degrees from the outside circumference of the curved tube.

10. The tracheostomy device of claim 9, wherein said beveled edge is angled at 30 degrees from the outside circumference of the curved tube.

11. A tracheostomy kit comprising an instruction manual, a tracheostomy tube and a tracheostomy device of any preceding claim.
